# EUROPEAN PATENT APPLICATION

(11) **EP 2 949 653 A1**
(43) Date of publication of application: **02.12.2015**
(21) Application number: 15169064.1
(22) Date of filing: 25.05.2015
(51) Int. Cl.: C07D 413/12, A61K 31/42, A61K 31/4439, A61K 31/4709, C07D 261/18

(54) **ISOXAZOLE DERIVATIVES AS LEUKOTRIENE BIOSYNTHESIS INHIBITORS**

(30) Priority: 27.05.2014 TR 201405943
(71) Applicant: Banoglu, Erden, 06330 Ankara (TR); Caliskan, Burcu, 06330 Ankara (TR); Werz, Oliver, 07743 Jena (DE)
(72) Inventor: BANOGLU, Erden, 06330 Ankara (TR); CALISKAN, Burcu, 06330 Ankara (TR); WERZ, Oliver, 07743 Jena (DE); EREN, Gokcen, 06330 ANKARA (TR); CELIKOGLU, Ersan, 06330 Ankara (TR); LEVENT, Serkan, 06330 Ankara (TR); VOELKER, Susanna, 07743 Jena (DE); GARSCHA, Ulrike, 07743 Jena (DE); ALTENHOFEN, Wolfram, 22457 Hamburg (DE)
(74) Representative: Cayli, Hülya

(57) **Abstract**

The present invention relates to isoxazole derivatives of formula (I) and their synthesis as inhibitors of leukotriene (LT) biosynthesis by modulating the activity of 5-lipoxygenase (5-LO) and 5-lipoxygenase-activating protein (FLAP). The present invention also relates to pharmaceutical compositions comprising these isoxazole derivatives, as well as, their use for treating leukotriene-related inflammatory disorders such as bronchial asthma, chronic obstructive pulmonary disease (COPD), allergic rhinitis, psoriasis, pruritus, arthritis, atherosclerosis, cancer and other inflammatory and autoimmune ailments.

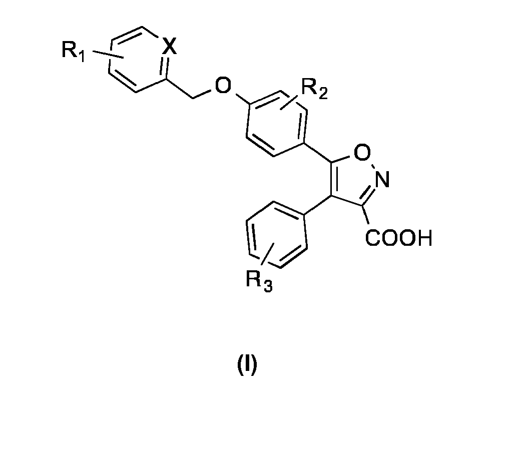

## Description

### Field of the Invention

This invention relates to novel substituted isoxazole-3-carboxylic acid compounds and their cellular leukotriene biosynthesis inhibitor activities.

### Background of the Invention

5-Lipoxygenase (5-LO) is a key enzyme involved in the biosynthesis of leukotrienes (LTs), which are lipid mediators of immune and inflammatory responses, with important roles in respiratory (asthma) diseases, cardiovascular diseases (CVD) (atherosclerosis), allergy, autoimmune diseases and certain types of cancer (prostate cancer, pancreatic cancer, breast cancer, hepatocellular carcinoma). 5-LO catalyzes the insertion of molecular oxygen into arachidonic acid (AA) to generate the unstable epoxide intermediate LTA₄, which is further metabolized to bioactive LTs by the enzymes LTA₄-hydrolase (produces LTB₄) or LTC₄-synthase (produces LTC₄) (Radmark, O. et al., Trends Biochem. Sci. 2007, 32, 332). LTC₄ is further metabolized to LTD₄ and then to LTE₄ which are collectively named cysteinyl(cys)-LTs.

Substantial experimental evidence indicates that 5-LO-mediated LT biosynthesis is facilitated by a helper protein, namely 5-LO-activating protein (FLAP), which may play a role as a scaffold protein for the transfer of AA to 5-LO *(*Abramovitz, M. et al., Eur. J. Biochem. 1993, 215(1), 105*;* Ferguson, A. D. et al., Science 2007, 317, 510*).* LTs are produced by a variety of cell types including neutrophils and monocytes (mainly generates LTB₄), macrophages and mast cells, basophils and bronchial epithelial cells (produces cys-LTs). LTB₄ acts as a high affinity ligand for BLT₁ and BLT₂ receptors, belonging to the family of G protein-coupled receptors (GPCRs), which stimulates the inflammatory response as well as neutrophil and eosinophil chemotaxis whereas the cys-LTs activate other GPCRs such as CysLT₁ and CysLT₂ resulting bronchoconstriction, airway edema, mucus secretion, increased vascular permeability and eosinophil recruitment (Peters-Golden, M. et al., N. Engl. J. Med. 2007, 357(18), 1841).

Two major pharmacological approaches pursued for the development of anti-LT therapy: (I) antagonism of cys-LTs at CysL TR₁ and (II) inhibition of the biosynthesis of 5-LO-mediated LT products (i.e., cys-LTs, LTB4 and 5-H(p)ETE) (Werz, O. and Steinhilber, D., Pharmacol. Ther. 2006, 112(3), 701*).* However, while CysL TR₁ antagonists (e.g., montelukast) are successfully applied in asthma therapy, the development of LT biosynthesis inhibitors is less advanced. So far, the direct 5-LO inhibitor zileuton is the only marketed LT synthesis inhibitor, but it is not widely prescribed due to significant side effects, poor pharmacokinetics, and multiple dosing. Recently, a new 5-LO inhibitor (MK-0633, setileuton) was reported to be under investigation for the treatment of asthma and atherosclerosis *(*Ducharme, Y. et al., ACS Med. Chem. Lett. 2010, 1, 170-174*).*

Nevertheless, the intervention with LTs through 5-LO and/or FLAP inhibition may be advantageous over CysL TR₁ antagonists because of their interference with formation of all 5-LO-mediated products resulting in broad-spectrum anti-LT agents. Besides targeting 5-LO, inhibition of LT biosynthesis may also be achieved by targeting FLAP (Evans, J. F. et al., Trends Pharmacol. Sci. 2008, 29, 72). A number of FLAP inhibitors have been reported in the scientific literature (see for example Hutchinson, J. H. et al., J. Med. Chem. 2009, 52, 5803*;* Stock, N. et al., Bioorg. Med. Chem. Lett. 2010, 20, 213*;* Bioorg. Med. Chem. Lett. 2010, 20, 4598; J. Med. Chem. 2011, 54, 8013*)* and in US. Patents (e.g., US2012/0277226A1, US 8,420,655B2, US 2007/0123522A1, US 2007/0219206A1, US 2007/0225285A1). Early candidates like MK-886, BAY X1005 and MK-591 that all inhibit LT synthesis by targeting FLAP underwent phase I and II studies and demonstrated clinical benefits in allergic asthma trials but were not further developed. Nevertheless, the recently developed FLAP antagonists that are all based on the structure of the indole-type FLAP inhibitor MK-886 such as AM803 (GSK2190915), AM643, and AM103 (GSK2190914) with slight structural modifications were shown to be efficacious in preclinical studies of inflammatory diseases as well as in phase II trials with patients suffering from asthma and are currently under further clinical investigations, however no FLAP inhibitor has yet reached the market.

Meanwhile, an isoxazole derivative with moderate anti-leukotriene activity has been published, designated as "compound A" (Banoglu et al. Bioorg. Med. Chem. 2012, 20, 3728). Compound A however exhibits a rather weak anti-leukotriene activity with IC₅₀ value of 4.4 µM in a cell-based assay and acts as a direct 5-LO inhibitor by equally well suppressing LTB₄ biosynthesis both in intact and cell-free assays.

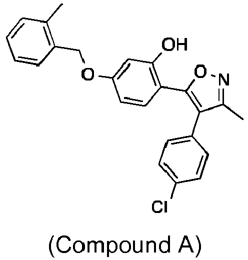

### Brief Description of the Invention

Present invention is related to a compound of the following formula I 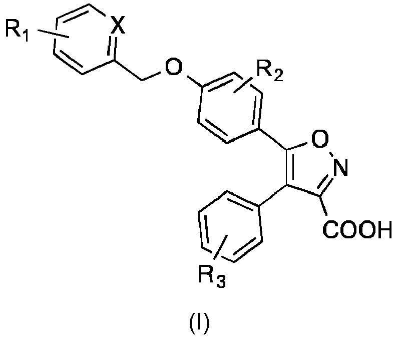 wherein,
R₁ is selected from the group consisting of hydrogen, halogen, hydroxy, C₁-C₅ alkoxy, C₁-C₅ alkyl, CF₃, nitro, CN, a condensed aromatic ring;
R₂ is selected from the group consisting of hydrogen, halogen, hydroxy, C₁-C₅ alkoxy, C₁-C₅ alkyl, CF₃, nitro, CN;
R₃ is selected from the group consisting of hydrogen, halogen, C₁-C₅ alkyl, hydroxy, C₁-C₅ alkoxy, CF₃, nitro, CN, amino;
X is C or N,
and/or pharmaceutically acceptable salts and/or solvates thereof. Present invention also is related to the use of these compounds as a medicament for human or animals in the treatment or prevention of ailments which are responsive to the inhibition of leukotriene biosynthesis selected from the group of respiratory diseases like asthma, chronic obstructive pulmonary disease, cardiovascular diseases like atherosclerosis, myocardial infarction, stroke, major adverse cardiac events (MACE), allergic diseases like rhinitis, pruritus, rhinorrhea, sneezing, autoimmune diseases and cancer.

### Figures

Figure 1 shows the inhibition of 5-LO mediated product formation by 4-(4-chlorophenyl)-5-(4-(quinolin-2-ylmethoxy)phenyl) isoxazole-3-carboxylic acid (compound 9hj) in human neutrophils stimulated with Ca-ionophore A23187 (2.5 µM).
Figure 2 shows the inhibition of 5-LO mediated product formation in human neutrophils stimulated with Ca-ionophore A23187 (2.5 µM), with the addition of 80 µM exogenous arachidonic acid (AA).
Figure 3 shows the inhibition of 5-LO mediated product synthesis by compound 9hj in intact human monocytes stimulated with A23187 (2.5 µM) in absence of exogenous AA.
Figure 4A shows inhibitory activity of Compound 9hj on 5-LO mediated product synthesis in human neutrophils in the presence of cell-stress (0.3 M NaCl).
Figure 4B shows inhibitory activity of Compound 9hj on 5-LO mediated product synthesis in human neutrophils in the presence of cell-stress (0.25 mM diamide).
Figure 5 shows the inhibitory activity of 9hj on the activity of human recombinant 5-LO for 5-LO mediated product formation in the presence of 3, 10 and 30 µM substrate AA.
Figure 6 shows the inhibitory activity of Compound 9hj on 5-LO in neutrophil homogenates with or without 1 mM dithiothreitol (DTT).
Figure 7 shows the inhibition of 5-LO mediated product synthesis by Compound 9hj in stably transfected HEK cells expressing mutated 5-LO and 5-LO plus FLAP proteins.
Figure 8 shows the analysis of 5-LO subcellular localization by immunofluorescense microscopy (IFM).
Figure 9 shows the inhibitory activity of Compound 9hj on zymosan-induced peritonitis in mice.

### Object of the Invention

One objective of the present invention is to provide novel compounds which are strong inhibitors of cellular leukotriene biosynthesis and compositions thereof.

Other object of the invention is to provide the following compound; 4-(4-chlorophenyl)-5-(4-(quinolin-2-ylmethoxy)phenyl)isoxazole-3-carboxylic acid (hereinafter referred to as compound 9hj).

Another objective of the present invention is to provide compounds and medicaments for human or animals for use in the treatment or prevention of ailments which are responsive to the inhibition of leukotriene biosynthesis selected from the group of respiratory diseases like asthma, chronic obstructive pulmonary disease, cardiovascular diseases like atherosclerosis, myocardial infarction (MI), stroke, major adverse cardiac event (MACE), allergic diseases like rhinitis, pruritus, rhinorrhea, sneezing, autoimmune diseases and cancer like prostate cancer, pancreatic cancer, breast cancer, hepatocellular carcinoma.

### Detailed Description of the Invention

The present invention relates to compounds bearing the structure shown in Formula 1, 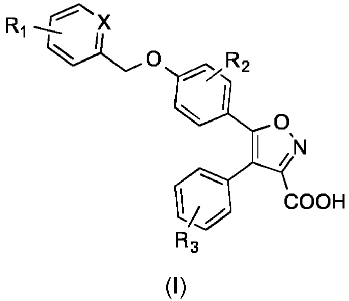 pharmaceutically acceptable salts or pharmaceutically acceptable solvates thereof, wherein,
R₁ is selected from the group consisting hydrogen, halogen, hydroxy, C₁-C₅ alkoxy, C₁-C₅ alkyl, CF₃, nitro, CN, a condensed aromatic ring;
R₂ is selected from the group consisting hydrogen, halogen, hydroxy, C₁-C₅ alkoxy, C₁-C₅ alkyl, CF₃, nitro, CN;
R₃ is selected from hydrogen, halogen, C₁-C₅ alkyl, hydroxy, C₁-C₅ alkoxy, CF₃, nitro, CN, amino;
X is C or N;

In another embodiment, the compounds of the present invention may be the following, for example:
Ethyl 4-(4-chlorophenyl)-5-(4-((2-methylbenzyl)oxy)phenyl)isoxazole-3-carboxylate (Compound no: 7aj);
Ethyl 4-(4-chlorophenyl)-5-(4-((3-fluorobenzyl)oxy)phenyl)isoxazole-3-carboxylate (Compound no: 7bj);
Ethyl 4-(4-chlorophenyl)-5-(4-((4-methylbenzyl)oxy)phenyl)isoxazole-3-carboxylate (Compound no: 7cj);
Ethyl 4-(3-chlorophenyl)-5-(4-((2-methylbenzyl)oxy)phenyl)isoxazole-3-carboxylate (Compound no: 8aj);
Ethyl 4-(3-chlorophenyl)-5-(4-((3-fluorobenzyl)oxy)phenyl)isoxazole-3-carboxylate (Compound no: 8bj);
4-(4-Chlorophenyl)-5-(4-((2-methylbenzyl)oxy)phenyl)isoxazole-3-carboxylic acid (Compound no: 9aj);
4-(4-Chlorophenyl)-5-(4-((3-fluorobenzyl)oxy)phenyl)isoxazole-3-carboxylic acid (Compound no: 9bj);
4-(4-Chlorophenyl)-5-(4-((4-methylbenzyl)oxy)phenyl)isoxazole-3-carboxylic acid (Compound no: 9cj);
4-(4-Chlorophenyl)-5-(4-((3-cyanobenzyl)oxy)phenyl)isoxazole-3-carboxylic acid (Compound no: 9dj);
5-(4-((2-chlorobenzyl)oxy)phenyl)-4-(4-chlorophenyl)isoxazole-3-carboxylic acid (Compound no: 9ej);
4-(4-Chlorophenyl)-5-(4-((2-(trifluoromethyl)benzyl)oxy)phenyl)isoxazole-3-carboxylic acid (Compound no: 9fj);
4-(4-Chlorophenyl)-5-(4-(pyridin-2-ylmethoxy)phenyl)isoxazole-3-carboxylic acid (Compound no: 9gj);
4-(4-Chlorophenyl)-5-(4-(quinolin-2-ylmethoxy)phenyl)isoxazole-3-carboxylic acid (Compound no: 9hj);
4-(4-Chlorophenyl)-5-(2-methyl-4-((4-methylbenzyl)oxy)phenyl)isoxazole-3-carboxylic acid (Compound no: 9ck);
4-(4-Chlorophenyl)-5-(4-((3-cyanobenzyl)oxy)-2-methylphenyl)isoxazole-3-carboxylic acid (Compound no: 9dk);
5-(4-((2-Chlorobenzyl)oxy)-2-methylphenyl)-4-(4-chlorophenyl)isoxazole-3-carboxylic acid (Compound no: 9ek);
4-(4-Chlorophenyl)-5-(2-methyl-4-((2-(trifluoromethyl)benzyl)oxy)phenyl)isoxazole-3-carboxylic acid (Compound no: 9fk);
4-(4-Chlorophenyl)-5-(2-methyl-4-(pyridin-2-ylmethoxy)phenyl)isoxazole-3-carboxylic acid (Compound no: 9gk);
4-(4-Chlorophenyl)-5-(2-methyl-4-(quinolin-2-ylmethoxy)phenyl)isoxazole-3-carboxylic acid (Compound no: 9hk);
4-(3-Chlorophenyl)-5-(4-((2-methylbenzyl)oxy)phenyl)isoxazole-3-carboxylic acid (Compound no: 10aj);
4-(3-Chlorophenyl)-5-(4-((3-fluorobenzyl)oxy)phenyl)isoxazole-3-carboxylic acid (Compound no: 10bj);
4-(3-Chlorophenyl)-5-(4-((3-fluorobenzyl)oxy)-2-methylphenyl)isoxazole-3-carboxylic acid (Compound no: 10bk);

Compounds, disclosed herein, may be prepared, for example, by techniques well known in organic synthesis and familiar to a practitioner ordinarily skilled in art of this invention. In addition, the processes, described herein, may enable the synthesis of compounds of the present invention. Further, the various steps outlined in below Scheme may be used to prepare the compounds of present invention.

In summary, the aryloxyacetophenones **(3aj-hj** or **3bk-hk)** were made by the reaction of commercially available benzyl bromides, 2-(chloromethyl)pyridine or 2-(chloromethyl)quinoline **(1a-h)** and 4-hydroxyacetophenones **(2j-k),** and were further used to generate intermediate enolates **(4aj-hj or 4bk-hk)** by reaction with dimethyl oxalate.

This keto-enol ester was later reacted with hydroxylamine hydrochloride to give the isoxazole intermediates **(5aj-hj** or **5bk-hk).** Subsequently, bromination at C(4)-pyrazole was made by bromine in acetic acid **(6aj-hj** or **6bk-hk).** Later, the intermediates of this class were cross-coupled with phenyl boronate esters having the m- or *p*-halogen substituent to give ethyl isoxazole-3-carboxylates **(7aj-hj, 7ck-hk, 8aj, bj, bk),** which subsequently hydrolyzed to the final isoxazole-3-carboxylic acids of the present invention **(9aj-hj, 9ck-hk, 10aj, bj, bk)** (Scheme 1, Table 1).

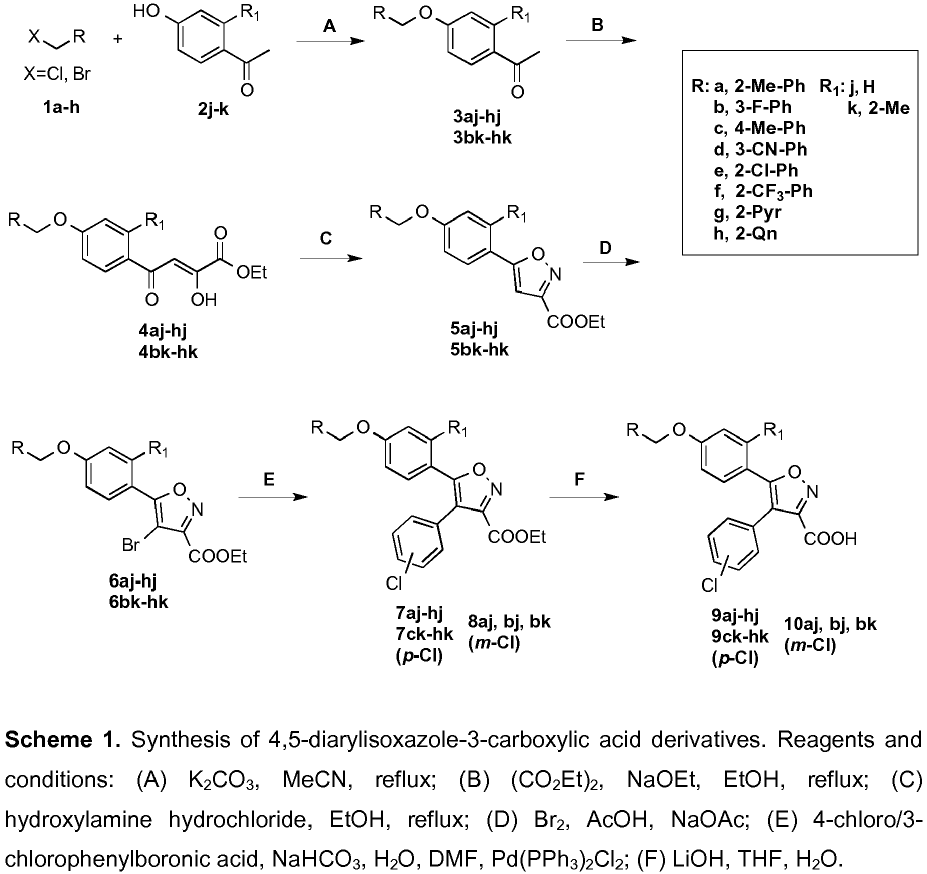

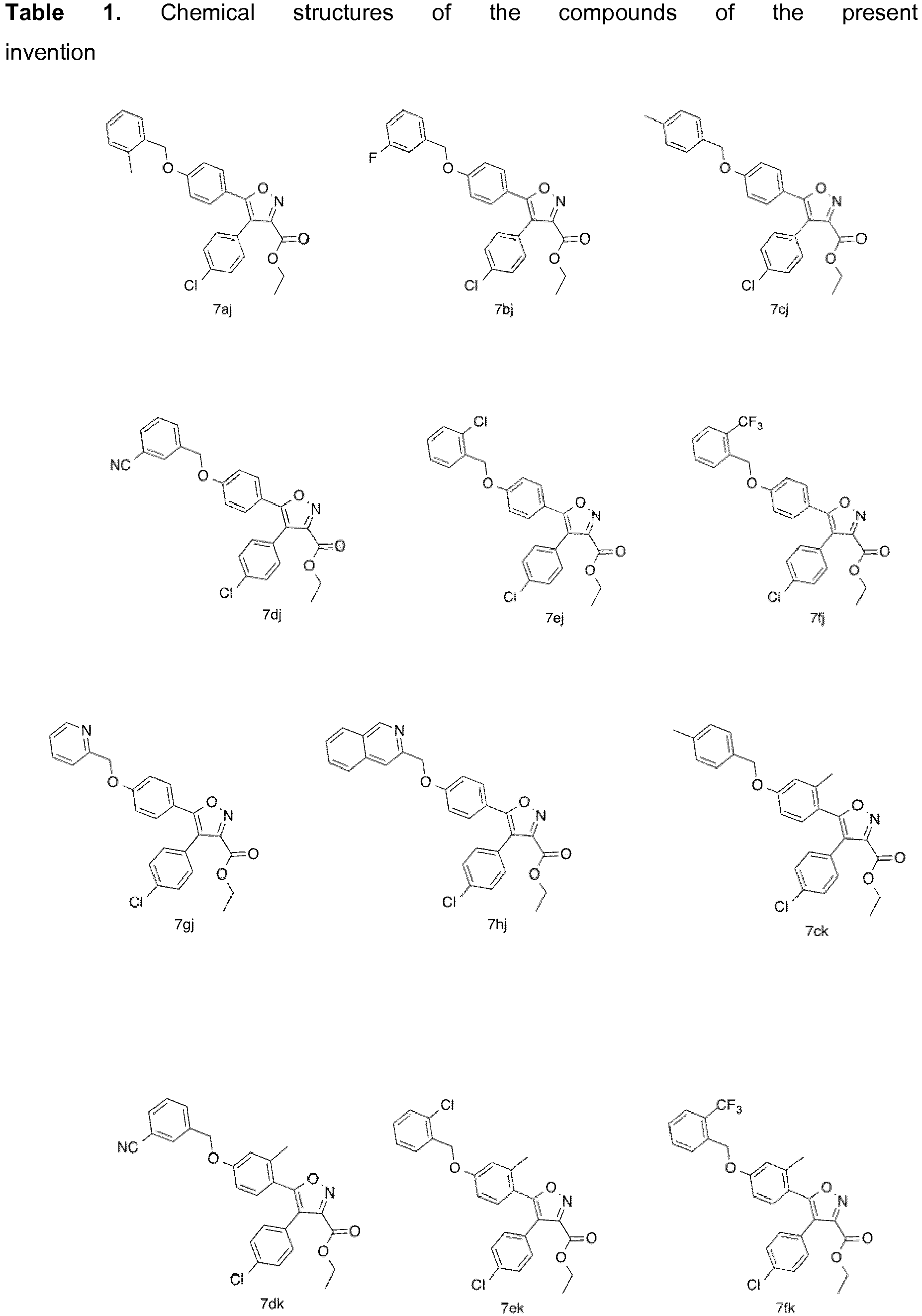

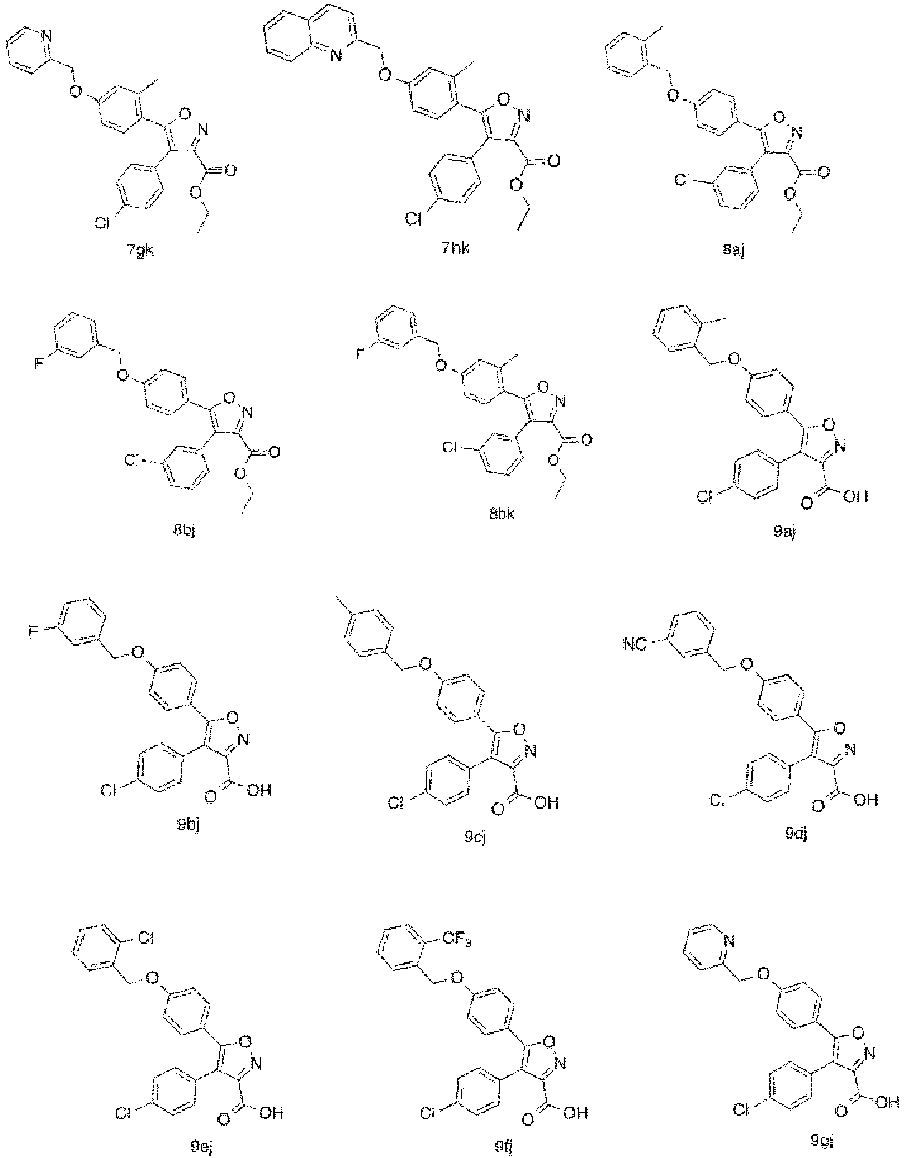

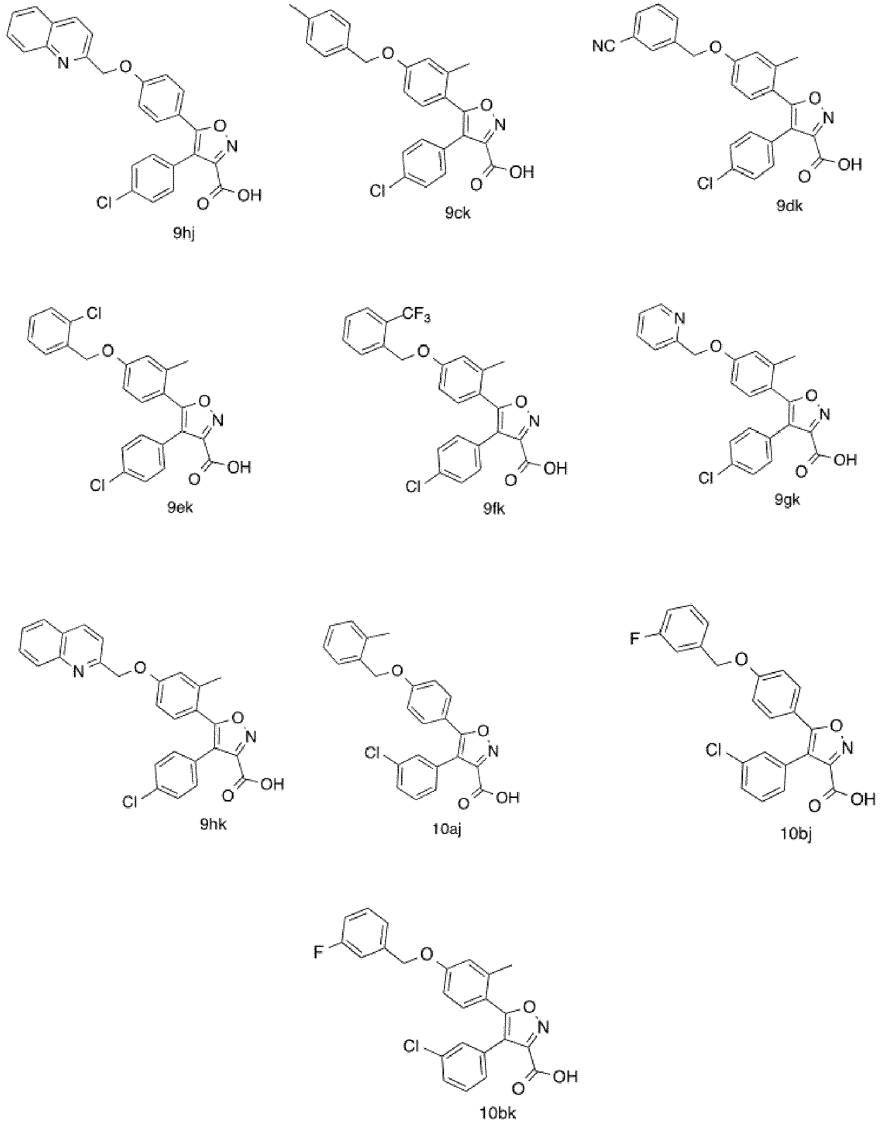

The present invention preferably includes compounds, which efficiently intervene with cellular LT biosynthesis (Table 2). They may target the 5-LO enzyme but not limited to 5-LO as a target. Compounds also preferably inhibit the cellular LT biosynthesis without a significant effect on purified 5-LO under cell free conditions, however presumably targeting FLAP. Such preferred activity may indicate an ability to reduce the incidence of aforementioned inflammatory pathologies. The detailed results of the biological studies are described in the following.

Cell based and cell-free assays carried out in biological studies, immunofluorescence microscopy analysis and zymosan-induced peritonitis in mice are performed as described hereinabove (Pegola, C. and et al., British J. Pharmacology, 2014, D01:10.1111/bph.12625). In the cell based assays (intact neutrophils or monocytes), 5-LO mediated product formation is started, after pre-incubation with test compounds (or control DMSO) for 15 min. at 37 °C, with addition of Ca-ionophore A23187 (2.5 µM) to the fresh 5 x 10⁶ neutrophils or monocytes suspended in 1 ml of PGC buffer (phosphate buffered saline (PBS) solution containing 1 mg ml⁻¹ glucose and 1mM CaCl₂, pH 7.4). After the incubation for 10 min. at 37 °C, the reaction is ended with addition of 1 ml of methanol, and 30 µL 1 N HCl, 200 ng prostaglandin B₁ and 500 µL PBS are added to the mixture. In cell-free assays (isolated 5-LO and cell homogenates), human recombinant 5-LO protein or 40,000g supernatant is added to 1 ml of 5-LO reaction mixture (PBS, pH 7.4, 1 mM EDTA, 1 mM ATP), and then incubated with test compounds (or control DMSO) for 10 min. at 4°C, heated to 37 °C and 2 mM CaCl₂ as well as AA at certain concentrations are added. The reaction is ended as described for the cell-based assays. Resulting 5-LO mediated products are extracted and analysed by the known method of high pressure liquid chromatography (HPLC) (Pergola, C. Proc Natl. Acad. Sci. U.S.A. 2008, 105, 19881). 5-LO mediated product formation (LTB₄ and all trans isomers, and 5 (S)-hydro(pero)xy-6-trans-8, 11, 14-cis-eicosatetraenoic acid, 5-H(p)ETE) is expressed as ng per 10⁶ cell. Cysteinyl-LTs have not been analyzed because that the amount of C₄, D₄ and E₄ and LTB₄ oxidation products are below the detection limit.

The problem solved in the present invention is to produce novel LT biosynthesis inhibitors with unexpected activity profile by modification of isoxazole compound A, previously discovered by our group through virtual screening against FLAP pharmacophore model, which inhibits cellular LT biosynthesis by directly interfering with 5-LO (IC₅₀PMNL=4.4 µM; IC₅₀cell-free=1.9 µM) but not with FLAP (Banoglu, E. and et al. Bioorg. Med. Chem. 20, 3728, 2012)*.* Unexpectedly, the removal of the 2-hydroxyl group on the 5-phenyl substituent of the central isoxazole of compound A causes a complete loss of activity for inhibition of cellular LT biosynthesis. Surprisingly, conversion of C(3)-methyl of isoxazole in Compound A (without 2-hydroxyl substituent) to a C(3)-carboxyl recovered the inhibition of cellular LT biosynthesis by directly targeting 5-LO (compounds 9aj-gj, 9dk, 10aj-bj and 10bk). Again, unpredictable by a skilled person in the art, the introduction of a methyl group in the carboxylic acid series at the same place of hydroxyl in compound A, caused a significant or complete loss of direct 5-LO inhibitory activity (9ck-hk, IC₅₀cell-free= 5 - >30 µM) although the potency for suppressing cellular LT biosynthesis is still maintained (IC₅₀PMNL=0.8 to 2.0 µM), suggesting that these compounds may inhibit cellular LT biosynthesis acting on a different target other than 5-LO, most preferably on FLAP.

One other exceptional result in this study was obtained with one of the compounds (9hj), which demonstrated rather weak activity for inhibition of purified 5-LO under cell-free conditions (IC₅₀cell-free= 8 µM) but showed potent inhibition of LT biosynthesis in intact neutrophils stimulated by Ca-ionophore A23187 (IC₅₀PMNL= 0.24 µM) (Table 2).

**Table 2. Effects of test compounds on 5-LO mediated product synthesis in cell-based (intact neutrophils) and in cell-free (purified 5-LO) assays**

| Cmpd. | 5-LO mediated product formation in intact neutrophils (PMNL)^{a} (% of control) | | Inhibition of purified 5-LO activity IC₅₀ (µM)^{b} (remaining activity at 30 µM [%])^{c} | |
|---|---|---|---|---|
| | at 10 µM | at 1 µM | cell based | cell-free |
| (Compound A) | 3.0 ± 0.7 | 66.7 ± 11.5 | 4.42 ± 1.13 | 1.90 ±0.46 |
| 7aj | 71.9 ± 10.3 | 85.2 ± 0.7 | n.d. | n.d. |
| 7bj | 55.3 ± 12.1 | 66.1 ± 14.3 | n.d. | n.d. |
| 7cj | 61.4 ± 12.4 | 78.3 ± 16.2 | n.d. | n.d. |
| 8aj | 83.4 ± 16.2 | 88.0 ± 10.2 | n.d. | n.d. |
| 8bj | 80.8 ± 11.8 | 90.6 ± 11.5 | n.d. | n.d. |
| **9aj** | **13.1 ± 3.9** | **74.5 ± 8.6** | **1.70 ± 0.47** | **0.48 ± 0.03** |
| **9bj** | **19.6 ± 9.5** | **52.3 ± 8.3** | **1.29 ± 0.61** | **0.38 ± 0.04** |
| **9cj** | **15.74 ± 4.53** | **55.81 ± 4.31** | **1.40 ± 0.09** | **5.94 ± 0.36** |
| **9dj** | **6.91 ± 3.92** | **70.05 ± 9.14** | **4.13 ± 0.33** | **16.32 ± 5.95** |
| **9ej** | **5.10 ± 1.46** | **65.68 ± 5.90** | **1.20 ± 0.23** | **6.11 ± 0.90** |
| **9fj** | **5.50 ± 2.21** | **52.81 ± 5.94** | **0.77 ± 0.19** | **6.04 ± 0.72** |
| **9gj** | **21.26 ± 8.34** | **82.08 ± 15.25** | **4.09 ± 0.12** | **7.39 ± 2.45** |
| **9hj** | **2.73 ± 1.41** | **12.12 ± 5.26** | ***0.24*±*0.07*** | **8.01 ± 3.03** |
| **9ck** | **2.26 ± 1.86** | **50.95 ± 6.68** | **1.20 ± 0.11** | **> 30^{c} (53±13%)** |
| **9dk** | **0.42 ± 0.21** | **77.43 ± 13.24** | **2.36 ± 0.32** | **5.01 ± 1.38** |
| **9ek** | **2.86 ± 2.59** | **54.87 ± 6.46** | **1.45 ± 0.09** | **> 30^{c} (51±11%)** |
| **9fk** | **3.48 ± 3.05** | **43.96 ± 10.30** | **0.82 ± 0.17** | **> 30^{c} (62±18%)** |
| **9gk** | **0.45 ± 0.45** | **73.23 ± 7.42** | **1.81 ± 0.11** | **> 30^{c} (73±15%)** |
| 9hk | 44.24 ± 15.69 | 69.14 ± 5.53 | 9.07 ± 1.98 | > 30^{c} (75±12%) |
| **10aj** | **0.7 ± 0.7** | **84.5 ± 9.1** | **2.08 ± 0.33** | **0.50 ± 0.03** |
| **10bj** | **8.4 ± 5.1** | **66.7 ± 8.5** | **1**.**64 ± 0**.**11** | **0.38 ± 0.03** |
| **10bk** | **9.8 ± 3.6** | **48.6 ± 7.9** | **0.82±0.19** | **1.12 ± 0.14** |

Compounds were tested in intact human neutrophils stimulated with 2.5 µM A23187 under cell based conditions and on purified 5-LO under cell free conditions. ^{a}Data are given as remaining activity as a percentage of control (100%) at 10 µM or 1 µM inhibitor concentrations (means ± SE, n = 3-6). The 100% value of the control [0.3% DMSO (dimethylsulfoxide), vehicle] corresponds to an average of 44 ng 5-LO mediated products per 10⁶ neutrophils. ^{b}The IC₅₀ values are given as mean ± SE of *n* = 3-4 determinations in duplicates.^{c}IC₅₀ > 30 µM; the remaining 5-LO activity (percentage of uninhibited control) at 30 µM of the test compound is given in parenthesis. n.d. means "not determined".

Compound 9hj of the present invention exhibited remarkable inhibition of LT biosynthesis.

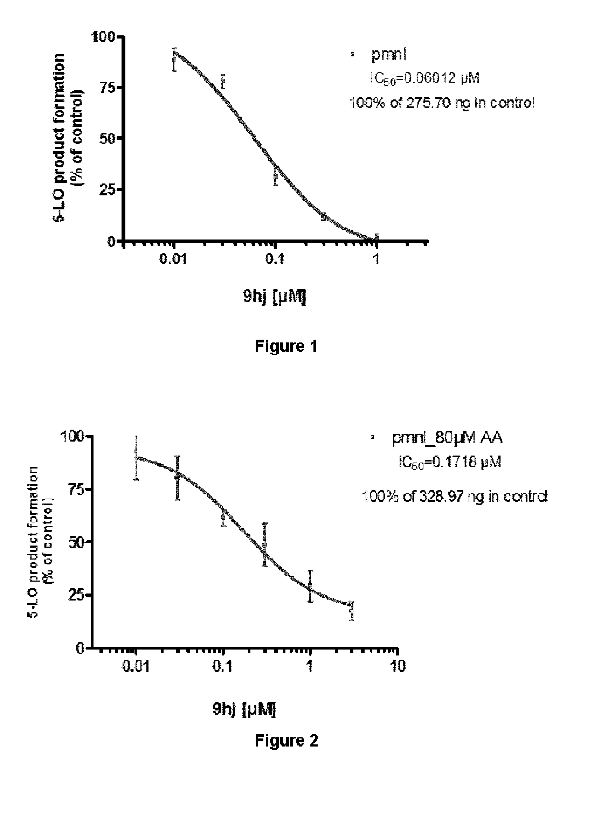

Compound 9hj inhibited 5-LO mediated product formation with IC₅₀ of 60 nM in human neutrophils. Neutrophils are stimulated with Ca-ionophore A23187 (2.5 µM), without the addition of exogenous arachidonic acid (AA), after pre-incubation with 9hj or vehicle (0.1 % DMSO). 100% controls correspond to 275.7 ng mL⁻¹ of 5-LO mediated products (Figure 1).

Compound 9hj inhibited 5-LO mediated product formation in human neutrophils stimulated with A23187 in the presence of 20 µM to 80 µM exogenous arachidonic acid with IC₅₀ of 170-350 nM, which is seen as very beneficial as compared to other FLAP inhibitors whose potencies are strongly reduced by addition of exogenous AA. Figure 2 shows inhibition of 5-LO mediated product formation in human neutrophils stimulated with Ca-ionophore A23187 (2.5 µM), in the presence of 80 µM exogenous arachidonic acid (AA), after pre-incubation with 9hj or vehicle (0.1% DMSO). 100% controls correspond to 328.97 ng mL⁻¹ of 5-LO mediated products.

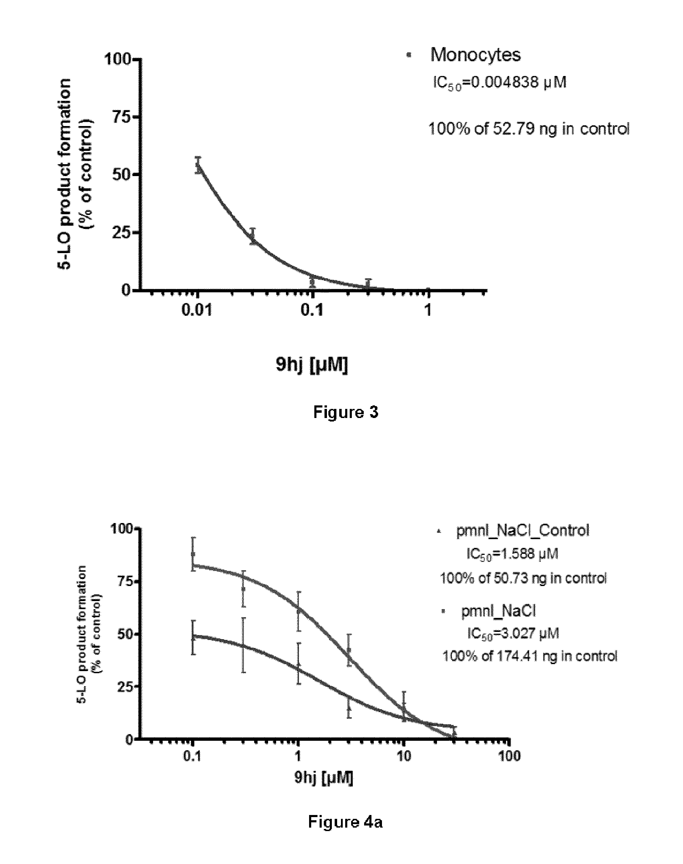

Compound 9hj inhibited 5-LO mediated product formation in human monocytes stimulated with A23187, with IC₅₀ of 5 nM. Figure 3 shows the effect of 9hj on 5-LO mediated product formation in intact human monocytes stimulated with A23187 (2.5 µM) in the absence of exogenous AA. Pre-incubation with 9hj or vehicle (0.1 % DMSO) was performed for 15 min at 37 °C for intact cells. 100% controls correspond to 52.79 ng mL⁻¹ of 5-LO mediated products.

Compound 9hj potently inhibited 5-LO mediated product synthesis in human neutrophils stimulated with lipopolysaccharide (LPS) plus N-formyl-methionyl-leucyl-phenylalanine (fMLP), which are more physiologically relevant test conditions than A23187 stimulation, with IC₅₀ of 10 nM.

Compound 9hj potently inhibited 5-LO mediated product synthesis in human monocytes stimulated with lipopolysaccharide (LPS) plus N-formyl-methionyl-leucyl-phenylalanine (fMLP), which are more physiologicallly relevant test conditions than A23187 stimulation, with IC₅₀ of 8 nM;

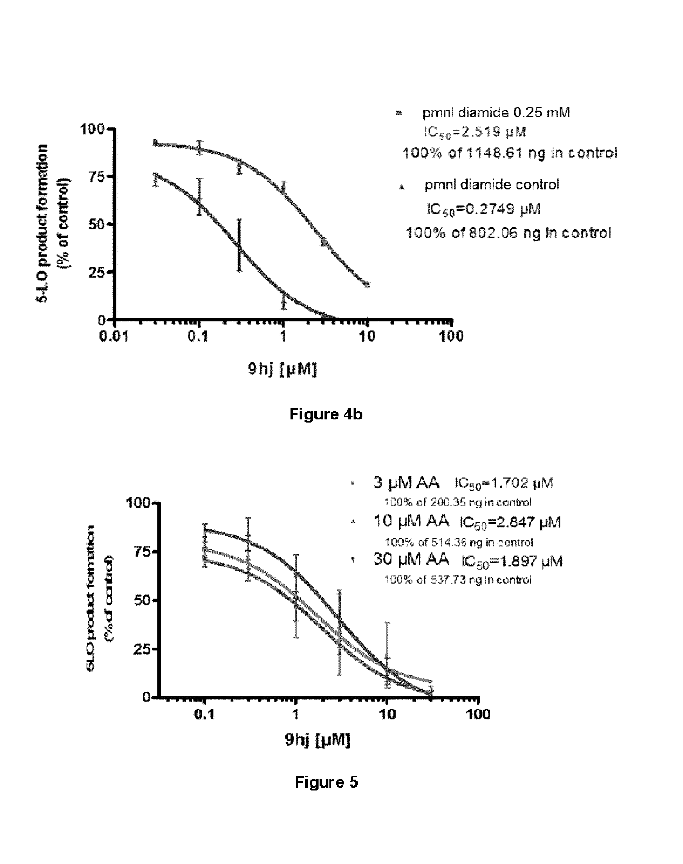

Inhibitory activity of compound 9hj on 5-LO mediated product synthesis in human neutrophils is shifted to the right by factor 10 in the presence of cell-stress (i.e., 0.3 M NaCl or 0.25 mM diamide), which is typical for non-redox type 5-LO inhibitors or FLAP inhibitors (Figure 4A-B). To intact neutrophils, 0.3 M sodium chloride (NaCl) or vehicle (DMSO) was added 3 min before addition of 9hj at 37°C. After addition of 9hj for another 10 min at 37°C, cells were stimulated with 20 µM AA for 10 min at 37°C. Data are expressed as percentage of vehicle control (0.1% DMSO). 100% controls correspond to 174.41 ng mL⁻¹ for 0.3 M NaCl and 50.73 ng mL⁻¹ for control (DMSO) (Figure 4A). To intact neutrophils, 0.25 mM diamide or vehicle (DMSO) was added 5 min before addition of 9hj at 37°C (Figure 4b). After addition of 9hj for another 15 min at 37°C, cells were stimulated with 2.5 µM A23187 plus 20 µM AA for 10 min at 37°C. Data are expressed as percentage of vehicle control (0.1% DMSO). 100% controls correspond to 1148.61 ng mL⁻¹ for 0.25 mM diamide and 802.06 ng mL⁻¹ for control (DMSO) (Figure 4B).

Compound 9hj inhibited the activity of isolated 5-LO at 3, 10 and 30 µM substrate AA concentrations, with a mean IC₅₀ of 2 µM, which shows that the 9hj is a non-competitive direct 5-LO inhibitor with low potency. Figure 5 shows the effects of 9hj on the activity of human recombinant 5-LO at different concentrations of AA for 5-LO mediated product formation. 9hj was added 15 min before addition of 2 mM Ca²⁺ plus 3 µM, 10 µM and 30 µM AA. 100% controls correspond to 200.35 ng mL⁻¹ for 3 µM AA, 514.36 ng mL⁻¹ for 10 µM AA and 537.73 ng mL⁻¹ for 30 µM AA.

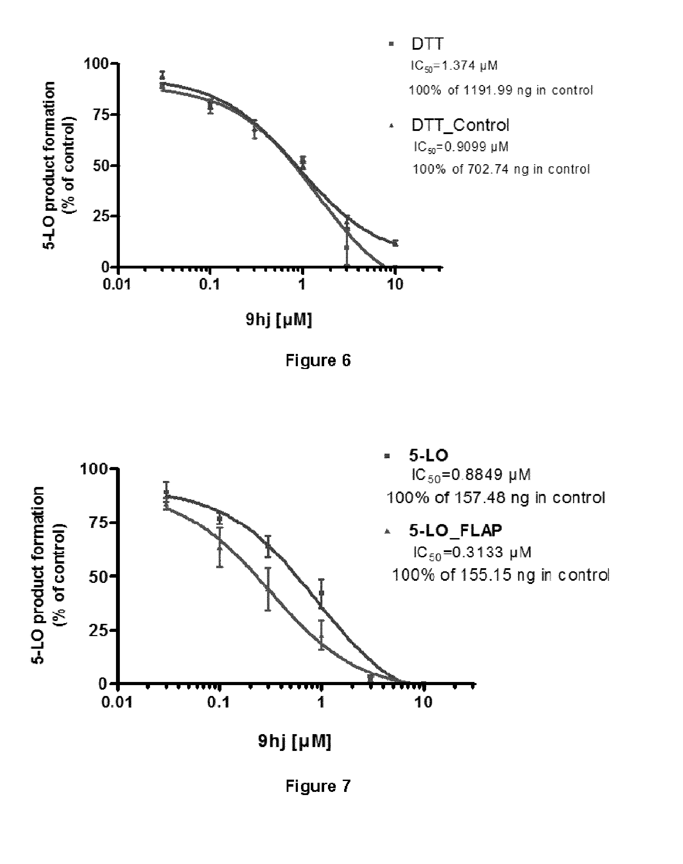

Supplementation of neutrophil homogenates with GSH (reducing conditions) does not affect 5-LO mediated product formation by 9hj (IC₅₀=1 µM) (Figure 6). GSH (1 mM) or vehicle (0.1% DMSO) was added to homogenates of neutrophils, prior to 9hj addition. After 10 min on ice, 1 mM CaCl₂ and 20 µM AA were added for 10 min at 37 °C. 100% controls correspond to 1191.99 ng mL⁻¹ for 1 mM DTT and 702.74 ng mL⁻¹ for control (0.1% DMSO).

Compound 9hj inhibited 5-LO mediated product synthesis in HEK293 cells transfected with only 5-LO, with IC₅₀ of 900 nM. Moreover, IC₅₀ value of the compound 9hj is 310 nM in the HEK293 cells transfected with 5-LO plus FLAP, which shows that FLAP induced increase in 5-LO mediated product synthesis is reduced effectively (Figure 7). Figure 7 shows the effect of 9hj on 5-LO mediated product formation in stably transfected HEK cells expressing mutated 5-LO and 5-LO plus FLAP proteins. After preincubation of cells with 9hj for 15 min at 37°C, cells were stimulated with 2.5 µM A23187 plus 3 µM AA for 10 min at 37°C. Data are expressed as percentage of vehicle control (0.1% DMSO). 100% controls correspond to 157.48 ng mL⁻¹ for 5-LO transfected cells and 155.15 ng mL⁻¹ for 5-LO plus FLAP transfected cells.

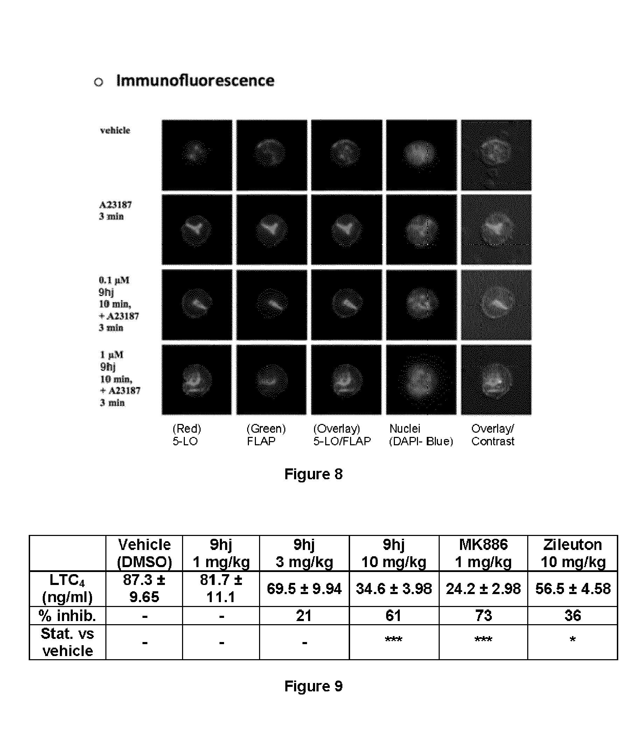

Compound 9hj inhibited translocation of 5-LO from soluble subcellular compartment to nuclear membrane in Ca-ionophore activated monocytes at the concentrations of 0.1 and 1 µM. Figure 8 shows the analysis of 5-LO localization by immunofluorescense microscopy (IFM). Neutrophils were preincubated with vehicle (0.1% DMSO), 9hj (0.1 and 1 µM), and stimulated by A23187 (2.5 µM; 3 min). Cells were fixed, permeabilized and incubated with mouse anti-5-LO and rabbit anti-FLAP, followed by Alexa Fluor 488 goat anti-rabbit IgG and Alexa Fluor 555 goat anti-mouse IgG. Red: 5-LO; green: FLAP; scale bar, 10 µm. The pictures shown are representative of three similar samples.

Compound 9hj was not cytotoxic to PMNL within 1 hour at 10 µM, and did not inhibit related enzymes, 12-LO or 15-LO, up to 10 µM in A23187-stimulated neutrophils.

Compound 9hj inhibited microsomal prostaglandin E synthase-1 (mPGES-1) with IC₅₀ of 5 µM.

Compound 9hj inhibited zymosan-induced peritonitis with ID₅₀ of 7 mg/kg (i.p.) dose (Figure 9). Male mice (n = 6, each group) were treated with 1, 3 and 10 mg kg⁻¹ 9hj, 1 mg kg⁻¹ MK886, 10 mg kg⁻¹ zileuton or vehicle (2% DMSO), 30 min before i.p. injection of zymosan. Analysis of LTC₄ levels in peritoneal exudate was performed 30 min after zymosan injection. Data are means ± SEM, n = 10-22. % inhibition: percentage of vehicle control. *p < 0.05; ***p < 0.001 vs vehicle (ANOVA_BONFERRONI).

Accordingly, compound 9hj of the present invention is a potent FLAP inhibitor (IC₅₀ = 8 and 10 nM, in monocytes and neutrophils, respectively) and also inhibits 5-LO uncompetitively (IC₅₀ = 1 µM) with good oral efficacy in vivo (ID₅₀ = 7 mg/kg). Based on the detailed pharmacology of the present invention, compound 9hj may represent a novel molecular structure that acts as a potent new-generation dual 5-LO/FLAP inhibitor, to be used for the treatment of LT-related disorders such as respiratory diseases (asthma, COPD, etc.), cardiovascular diseases (atherosclerosis, MI, stroke, MACE-major adverse cardiac events), allergic diseases (rhinitis, pruritus, etc.), autoimmune diseases (multiple sclerosis, rheumatoide arthritis, systemic lupus erythematosus etc.) and cancer (e.g. prostate cancer, pancreatic cancer, breast cancer, hepatocellular carcinoma).

The novel compound (I) of present invention or pharmaceutically acceptable salts or solvates thereof is able to be administered in a suitable pharmaceutical composition with at least one pharmaceutically acceptable carrier. Said composition is able to be administered by subcutaneous, intra-muscular, intravenous, oral, topical or any other technically known routes. Injectable formulations are able to be produced from sterile or sterilisable solution, or suspension or emulsion. The pharmaceutical composition administrable by oral route preferred in carrying out the present invention are solution, emulsion, suspension, powder, capsule, made of soft or hard gelatin, gastroresistant capsule, tablet, controlled release tablet, gastroresistant tablet or granulate. The composition is able to be prepared by methods known in the prior art.

## Claims

1. A compound of the following formula I 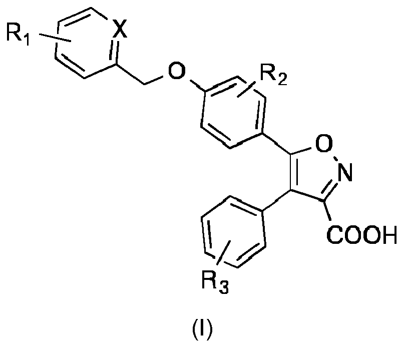 wherein,
R₁ is selected from the group consisting of hydrogen, halogen, hydroxy, C₁-C₅ alkoxy, C₁-C₅ alkyl, CF₃, nitro, CN, a condensed aromatic ring;
R₂ is selected from the group consisting of hydrogen, halogen, hydroxy, C₁-C₅ alkoxy, C₁-C₅ alkyl, CF₃, nitro, CN;
R₃ is selected from the group consisting of hydrogen, halogen, C₁-C₅ alkyl, hydroxy, C₁-C₅ alkoxy, CF₃, nitro, CN, amino;
X is C or N,
and/or pharmaceutically acceptable salts and/or solvates thereof.

2. A compound according to claim 1 wherein said compound is selected from:
Ethyl 4-(4-chlorophenyl)-5-(4-((2-methylbenzyl)oxy)phenyl)isoxazole-3-carboxylate;
Ethyl 4-(4-chlorophenyl)-5-(4-((3-fluorobenzyl)oxy)phenyl)isoxazole-3-carboxylate;
Ethyl 4-(4-chlorophenyl)-5-(4-((4-methylbenzyl)oxy)phenyl)isoxazole-3-carboxylate;
Ethyl 4-(3-chlorophenyl)-5-(4-((2-methylbenzyl)oxy)phenyl)isoxazole-3-carboxylate;
Ethyl 4-(3-chlorophenyl)-5-(4-((3-fluorobenzyl)oxy)phenyl)isoxazole-3-carboxylate;
4-(4-Chlorophenyl)-5-(4-((2-methylbenzyl)oxy)phenyl)isoxazole-3-carboxylic acid;
4-(4-Chlorophenyl)-5-(4-((3-fluorobenzyl)oxy)phenyl)isoxazole-3-carboxylic acid;
4-(4-Chlorophenyl)-5-(4-((4-methylbenzyl)oxy)phenyl)isoxazole-3-carboxylic acid;
4-(4-Chlorophenyl)-5-(4-((3-cyanobenzyl)oxy)phenyl)isoxazole-3-carboxylic acid;
5-(4-((2-chlorobenzyl)oxy)phenyl)-4-(4-chlorophenyl)isoxazole-3-carboxylic acid;
4-(4-Chlorophenyl)-5-(4-((2-(trifluoromethyl)benzyl)oxy)phenyl)isoxazole-3-carboxylic acid;
4-(4-Chlorophenyl)-5-(4-(pyridin-2-ylmethoxy)phenyl)isoxazole-3-carboxylic acid;
4-(4-Chlorophenyl)-5-(4-(quinolin-2-ylmethoxy)phenyl)isoxazole-3-carboxylic acid;
4-(4-Chlorophenyl)-5-(2-methyl-4-((4-methylbenzyl)oxy)phenyl)isoxazole-3-carboxylic acid;
4-(4-Chlorophenyl)-5-(4-((3-cyanobenzyl)oxy)-2-methylphenyl)isoxazole-3-carboxylic acid;
5-(4-((2-Chlorobenzyl)oxy)-2-methylphenyl)-4-(4-chlorophenyl)isoxazole-3-carboxylic acid;
4-(4-Chlorophenyl)-5-(2-methyl-4-((2-(trifluoromethyl)benzyl)oxy)phenyl)isoxazole-3-carboxylic acid
4-(4-Chlorophenyl)-5-(2-methyl-4-(pyridin-2-ylmethoxy)phenyl)isoxazole-3-carboxylic acid;
4-(4-Chlorophenyl)-5-(2-methyl-4-(quinolin-2-ylmethoxy)phenyl)isoxazole-3-carboxylic acid;
4-(3-Chlorophenyl)-5-(4-((2-methylbenzyl)oxy)phenyl)isoxazole-3-carboxylic acid;
4-(3-Chlorophenyl)-5-(4-((3-fluorobenzyl)oxy)phenyl)isoxazole-3-carboxylic acid;
4-(3-Chlorophenyl)-5-(4-((3-fluorobenzyl)oxy)-2-methylphenyl)isoxazole-3-carboxylic acid;
and/or pharmaceutically acceptable salts and/or solvates thereof.

3. A compound according to claim 1 wherein said compound is 4-(4-chlorophenyl)-5-(4-(quinolin-2-ylmethoxy)phenyl)isoxazole-3-carboxylic acid.

4. A compound according to claim 1 to 3 for use as a medicament.

5. A compound according to claim 1 to 3 for use as cellular leukotriene biosynthesis or 5-Lipoxygenase (5-LO)/ 5-Lipoxygenase-activating protein (FLAP) inhibitor.

6. A compound of the following formula I 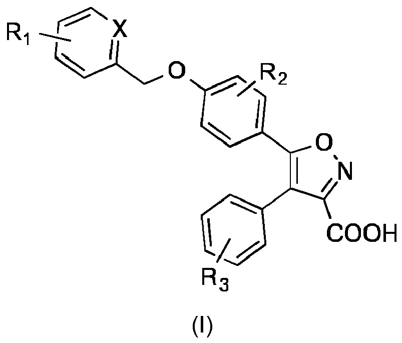 wherein,
R₁ is selected from the group consisting of hydrogen, halogen, hydroxy, C₁-C₅ alkoxy, C₁-C₅ alkyl, CF₃, nitro, CN, a condensed aromatic ring;
R₂ is selected from the group consisting of hydrogen, halogen, hydroxy, C₁-C₅ alkoxy, C₁-C₅ alkyl, CF₃, nitro, CN;
R₃ is selected from the group consisting of hydrogen, halogen, C₁-C₅ alkyl, hydroxy, C₁-C₅ alkoxy, CF₃, nitro, CN, amino;
X is C or N,
and/or pharmaceutically acceptable salts and/or solvates thereof for use in the treatment or prevention of ailments which are responsive to the inhibition of leukotriene biosynthesis selected from the group of respiratory diseases like asthma, chronic obstructive pulmonary disease, cardiovascular diseases like atherosclerosis, myocardial infarction, stroke, major adverse cardiac events (MACE), allergic diseases like rhinitis, pruritus, rhinorrhea, sneezing, autoimmune diseases and cancer, like prostate cancer, pancreatic cancer, breast cancer, hepatocellular carcinoma.

7. A compound according to claim 6 **characterized in that** said compound is selected from:
Ethyl 4-(4-chlorophenyl)-5-(4-((2-methylbenzyl)oxy)phenyl)isoxazole-3-carboxylate;
Ethyl 4-(4-chlorophenyl)-5-(4-((3-fluorobenzyl)oxy)phenyl)isoxazole-3-carboxylate;
Ethyl 4-(4-chlorophenyl)-5-(4-((4-methylbenzyl)oxy)phenyl)isoxazole-3-carboxylate;
Ethyl 4-(3-chlorophenyl)-5-(4-((2-methylbenzyl)oxy)phenyl)isoxazole-3-carboxylate;
Ethyl 4-(3-chlorophenyl)-5-(4-((3-fluorobenzyl)oxy)phenyl)isoxazole-3-carboxylate;
4-(4-Chlorophenyl)-5-(4-((2-methylbenzyl)oxy)phenyl)isoxazole-3-carboxylic acid;
4-(4-Chlorophenyl)-5-(4-((3-fluorobenzyl)oxy)phenyl)isoxazole-3-carboxylic acid;
4-(4-Chlorophenyl)-5-(4-((4-methylbenzyl)oxy)phenyl)isoxazole-3-carboxylic acid;
4-(4-Chlorophenyl)-5-(4-((3-cyanobenzyl)oxy)phenyl)isoxazole-3-carboxylic acid;
5-(4-((2-chlorobenzyl)oxy)phenyl)-4-(4-chlorophenyl)isoxazole-3-carboxylic acid;
4-(4-Chlorophenyl)-5-(4-((2-(trifluoromethyl)benzyl)oxy)phenyl)isoxazole-3-carboxylic acid;
4-(4-Chlorophenyl)-5-(4-(pyridin-2-ylmethoxy)phenyl)isoxazole-3-carboxylic acid;
4-(4-Chlorophenyl)-5-(4-(quinolin-2-ylmethoxy)phenyl)isoxazole-3-carboxylic acid;
4-(4-Chlorophenyl)-5-(2-methyl-4-((4-methylbenzyl)oxy)phenyl)isoxazole-3-carboxylic acid;
4-(4-Chlorophenyl)-5-(4-((3-cyanobenzyl)oxy)-2-methylphenyl)isoxazole-3-carboxylic acid;
5-(4-((2-Chlorobenzyl)oxy)-2-methylphenyl)-4-(4-chlorophenyl)isoxazole-3-carboxylic acid;
4-(4-Chlorophenyl)-5-(2-methyl-4-((2-(trifluoromethyl)benzyl)oxy)phenyl)isoxazole-3-carboxylic acid;
4-(4-Chlorophenyl)-5-(2-methyl-4-(pyridin-2-ylmethoxy)phenyl)isoxazole-3-carboxylic acid;
4-(4-Chlorophenyl)-5-(2-methyl-4-(quinolin-2-ylmethoxy)phenyl)isoxazole-3-carboxylic acid;
4-(3-Chlorophenyl)-5-(4-((2-methylbenzyl)oxy)phenyl)isoxazole-3-carboxylic acid;
4-(3-Chlorophenyl)-5-(4-((3-fluorobenzyl)oxy)phenyl)isoxazole-3-carboxylic acid;
4-(3-Chlorophenyl)-5-(4-((3-fluorobenzyl)oxy)-2-methylphenyl)isoxazole-3-carboxylic acid;
and/or pharmaceutically acceptable salts and/or solvates thereof.

8. A compound according to claim 6 wherein said compound is 4-(4-chlorophenyl)-5-(4-(quinolin-2-ylmethoxy)phenyl)isoxazole-3-carboxylic acid.

9. 4-(4-chlorophenyl)-5-(4-(quinolin-2-ylmethoxy)phenyl)isoxazole-3-carboxylic acid and/or pharmaceutically acceptable salts or solvates thereof for use as a medicament.

10. 4-(4-chlorophenyl)-5-(4-(quinolin-2-ylmethoxy)phenyl)isoxazole-3-carboxylic acid and/or pharmaceutically acceptable salts and/or solvates thereof according to claim 9 for use in the treatment or prevention of ailments which are responsive to the inhibition of leukotriene biosynthesis selected from the group of respiratory diseases like asthma, chronic obstructive pulmonary disease, cardiovascular diseases like atherosclerosis, myocardial infarction, stroke, major adverse cardiac events (MACE), allergic diseases like rhinitis, pruritus, rhinorrhea, sneezing, autoimmune diseases and cancer like prostate cancer, pancreatic cancer, breast cancer, hepatocellular carcinoma.

11. A pharmaceutical composition comprising therapeutically effective amount of the compound according to claim 1-3 and/or pharmaceutically acceptable salts and/or solvates thereof and at least one pharmaceutically acceptable carrier.
